(19) 

Europäisches
Patentamt
European
Patent Office
Office européen
des brevets

(11)  **EP 4 338 736 A1**

(12)  **EUROPEAN PATENT APPLICATION**
published in accordance with Art. 153(4) EPC

(43) Date of publication:
**20.03.2024  Bulletin 2024/12**

(21) Application number: **22807522.2**

(22) Date of filing: **12.05.2022**

(51) International Patent Classification (IPC):
**A61K 31/4965** (2006.01)      **A61P 31/12** (2006.01)
**A61P 31/14** (2006.01)

(52) Cooperative Patent Classification (CPC):
**A61K 31/4965; A61P 31/12; A61P 31/14**

(86) International application number:
**PCT/JP2022/020028**

(87) International publication number:
**WO 2022/239823 (17.11.2022 Gazette 2022/46)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **14.05.2021   JP 2021082834
18.06.2021   JP 2021101341
04.11.2021   JP 2021180064**

(71) Applicant: **FUJIFILM Toyama Chemical Co., Ltd.
Chuo-ku
Tokyo 104-0031 (JP)**

(72) Inventors:
• **KOMENO Takashi
Toyama-shi, Toyama 930-8508 (JP)**
• **FURUTA Yousuke
Toyama-shi, Toyama 930-8508 (JP)**

(74) Representative: **Wächtershäuser & Hartz
Patentanwaltspartnerschaft mbB
Weinstraße 8
80333 München (DE)**

(54)  **AGENT FOR TREATING INFECTIOUS DISEASES AGAINST MUTANT VIRUSES OF NOVEL CORONAVIRUS**

(57)   This agent for treating coronavirus infectious diseases caused by mutant viruses of SARS-CoV-2 contains, as an active ingredient, 6-fluoro-3-hydroxy-2-pyrazinecarboxamide or a salt thereof.

EP 4 338 736 A1

**Description**

**TECHNICAL FIELD**

The present invention relates to a therapeutic agent for coronavirus infection caused by a variant of novel coronavirus comprising 6-fluoro-3-hydroxy-2-pyrazinecarboxamide (nonproprietary name, favipiravir; hereinafter referred to as "Compound A") or a salt thereof as an active ingredient.

**BACKGROUND ART**

The novel coronavirus (SARS-CoV-2) first reported in China at the end of 2019 (Non Patent Document 1) is an RNA virus belonging to the family *coronaviridae,* order *nidovirales* (Non Patent Document 2). When infected with this virus, fever and acute respiratory symptoms including cough and dyspnea develop (Non Patent Document 1), and when the condition is exacerbated, pneumonia develops. As of May 12, 2021, more than 650,000 individuals had been positive for SARS-CoV-2 in Japan, and, of these, more than 10,000 had died (Non Patent Document 3).

Compound A is an antiviral drug developed by Fujifilm Toyama Chemical Co., Ltd. (formerly Toyama Chemical Co., Ltd.) and obtained the manufacturing and marketing authorization in Japan in March 2014 for an indication limited to "infections with novel or re-emerging influenza virus (only for infections showing no or inadequate responses to other antiinfluenza virus drugs)." Given the mechanism of action that Compound A is converted to a triphosphorylated form (T-705RTP) in the body and inhibits a viral RNA polymerase selectively, Compound A may be effective against RNA viruses other than influenza virus. In fact, reports have shown that Compound A is effective against Ebola virus, as well as RNA viruses belonging to families *Arenaviridae* and *Bunyaviridae* in vitro or in vivo (Non Patent Documents 4, 5, and 6).

SARS-CoV-2 continues to undergo gene mutations at various sites of its genome, and many variants have been reported (https://nextstrain.org/ncov/global, https://cov-lineages.org/). Variants having high infection efficiency and variants of concern about their impact on effect of vaccines have also emerged (https://www.cdc.gov/coronavirus/2019-ncov/cases-updates/variant-surveillance/variant-info.html).

Methods for treating coronavirus infection caused by these SARS-CoV-2 variants have not been established. Additionally, while an antiviral drug remdesivir has been approved in Japan for the treatment of COVID-19, resistance mutations to remdesivir have already been reported (Non Patent Documents 7, 8, and 9), and there are concerns about reduction or loss of drug effectiveness. Therefore, a novel therapeutic agent is demanded.

**PRIOR ART DOCUMENT**

**NON PATENT DOCUMENT**

> **Non Patent Document 1:** Wang C, Horby PW, Hayden FG, Gao GF. A novel coronavirus outbreak of global health concern. Lancet. 2020; 395:470-3.
>
> **Non Patent Document 2:** Coronaviridae Study Group of the International Committee on Taxonomy of Viruses. The species Severe acute respiratory syndrome-related coronavirus: classifying 2019-nCoV and naming it SARS-CoV-2. Nat Microbiol. 2020; 5:536-44
>
> **Non Patent Document 3:** Kokunai no hassei jokyo nado (in Japanese) (Situation of occurrences etc.) [Internet]. Tokyo: Ministry of Health, Labour and Welfare [updated 2021 May 12; cited 2021 May 13]. Available from: https://www.mhlw.go.jp/stf/covid-19/kokunainohasseijoukyou.html
>
> **Non Patent Document 4:** Gowen BB, Wong MH, Jung KH, Sanders AB, Mendenhall M, Bailey KW, et al. In vitro and in vivo activities of T-705 against arenavirus and bunyavirus infections. Antimicrob Agents Chemother. 2007; 51:3168-76
>
> **Non Patent Document 5:** Mendenhall M, Russell A, Smee DF, Hall JO, Skirpstunas R, Furuta Y, et al. Effective oral favipiravir (T-705) therapy initiated after the onset of clinical disease in a model of arenavirus hemorrhagic fever. PLoS Negl Trop Dis. 2011; 5:e1342
>
> **Non Patent Document 6:** Oestereich L, Ludtke A, Wurr S, Rieger T, Munoz-Fontela C, Gunther S. Successful treatment of advanced Ebola virus infection with T-705 (favipiravir) in a small animal model. Antiviral Res. 2014; 105:17-21
>
> **Non Patent Document 7:** Szemiel et al. In vitro evolution of Remdesivir resistance reveals genome plasticity of SARS-CoV-2. bioRxiv 2021. Available from https://doi.org/10.1101/2021.02.01.429199
>
> **Non Patent Document 8:** Martinot M, Jary A, Fafi-Kremer S, Leducq V, Delagreverie H, Garnier M et al. Remdesivir failure with SARS-CoV-2 RNA-dependent RNA-polymerase mutation in a B-cell immunodeficient patient with protracted Covid-19. Clin Infect Dis. 2020 [doi: 10.1093/cid/ciaa1474]

**Non Patent Document 9:** Agostini ML, Andres EL, Sims AC, Graham RL, Sheahan TP, Lu X et al. Coronavirus Susceptibility to the Antiviral Remdesivir (GS-5734) Is Mediated by the Viral Polymerase and the Proofreading Exoribonuclease. mBio. 2018; 9:e00221-18

## SUMMARY OF INVENTION

### TECHNICAL PROBLEM

An object of the present invention is to provide a therapeutic agent for infection that exhibits effect against a variant of novel coronavirus.

### SOLUTION TO PROBLEM

Under the above-described circumstances, the present inventors have found that Compound A or a salt thereof can treat infection caused by a variant of novel coronavirus, and accomplished the present invention.

According to the present invention, the following is provided.

[1] A therapeutic agent for coronavirus infection caused by a variant of SARS-CoV-2, comprising 6-fluoro-3-hydroxy-2-pyrazinecarboxamide or a salt thereof as an active ingredient.

[2] The therapeutic agent for coronavirus infection according to [1], wherein at least one of mutations in the genetic information of the Wuhan-Hu-1 strain has occurred in the gene coding for S protein in the variant.

[3] The therapeutic agent for coronavirus infection according to [1], wherein at least one of mutations in the genetic information of the Wuhan-Hu-1 strain has occurred in the gene coding for M protein in the variant.

[4] The therapeutic agent for coronavirus infection according to [1], wherein at least one of mutations in the genetic information of the Wuhan-Hu-1 strain has occurred in the gene coding for E protein in the variant.

[5] The therapeutic agent for coronavirus infection according to [1], wherein at least one of mutations in the genetic information of the Wuhan-Hu-1 strain has occurred in the gene coding for N protein in the variant.

[6] The therapeutic agent for coronavirus infection according to [1], wherein at least one of mutations in the genetic information of the Wuhan-Hu-1 strain has occurred in the gene coding for an RNA-dependent RNA polymerase in the variant.

[7] The therapeutic agent for coronavirus infection according to [1], wherein at least one of mutations in the genetic information of the Wuhan-Hu-1 strain has occurred in the gene coding for an exonuclease in the variant.

[8] The therapeutic agent for coronavirus infection according to [1], wherein at least one of mutations in the genetic information of the Wuhan-Hu-1 strain has occurred in the gene coding for a protease in the variant.

[9] The therapeutic agent for coronavirus infection according to [1], wherein the variant is a virus with increased or decreased virulence as compared with that of SARS-CoV-2.

[10] The therapeutic agent for coronavirus infection according to [1], wherein the variant is a virus with increased or decreased human-human infection efficiency as compared with that of SARS-CoV-2.

[11] The therapeutic agent for coronavirus infection according to [1], wherein the variant is a virus with an increased immune escaping ability as compared with that of SARS-CoV-2.

[12] The therapeutic agent for coronavirus infection according to [1], wherein the variant is a virus with increased or decreased resistance to a coronavirus infection therapeutic drug as compared with that of SARS-CoV-2.

[13] The therapeutic agent for coronavirus infection according to [1], wherein the variant is a virus for which the effectiveness of a novel coronavirus vaccine has been increased or decreased as compared with for SARS-CoV-2.

[14] The therapeutic agent for coronavirus infection according to [1], wherein the variant is B.1.1.7 (501YV1), B.1.351 (501YV2), P.1 (501YV3), B.1.427 (20C/S:452R), B.1.429 (20C/S:452R), B.1.526 (20C/S:484K), B.1.526.1 (20C), B.1.525 (20A/S:484K), P.2 (20J), B.1.617 (20A), B.1.617.1 (20A/S:154K), B.1.617.2 (20A/S:478K), B.1.617.3 (20A), B.1.1.316, B.1.617 + S:V382L, or an experimentally or clinically isolated coronavirus resistant to remdesivir.

## ADVANTAGEOUS EFFECTS OF INVENTION

The therapeutic agent for infection of the present invention is useful for treatments such as therapeutic or prophylactic treatment of coronavirus infection caused by a variant of novel coronavirus.

## DESCRIPTION OF EMBODIMENTS

The present invention will be described in detail below.

In the present specification, each term has the following meaning, unless otherwise specified.

In this specification, a range of numerical values represented using "to" means a range including the numerical values before and after "to" as the minimum value and the maximum value, respectively.

Compound A means 6-fluoro-3-hydroxy-2-pyrazinecarboxamide.

Examples of a salt of Compound A include commonly known salts in basic groups such as an amino group or acidic groups such as a hydroxyl or carboxyl group.

Examples of salts in basic groups include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid, and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of salts in acidic groups include salts with alkali metals such as sodium and potassium; salts with alkaline earth metals such as calcium and magnesium; ammonium salts; and salts with nitrogen-containing organic bases such as trimethylamine, triethylamine, tributylamine, pyridine, *N,N*-dimethylaniline, N-methylpiperidine, N-methylmorpholine, diethylamine, dicyclohexylamine, procaine, dibenzylamine, *N*-benzyl-β-phenethylamine, 1-efenamine, *N,N'*-dibenzylethylenediamine, and meglumine.

Among the above-mentioned salts, preferred examples of salts include pharmacologically accepted salts, and more preferred examples of salts include salts with sodium or meglumine.

If Compound A or a salt thereof has isomers (e.g., optical isomers, geometric isomers, and tautomers), the present invention encompasses all these isomers, as well as hydrates, solvates, and all crystalline forms thereof.

The term "coronavirus" used in the present specification means an RNA virus belonging to family *coronaviridae* of order *Nidovirus.* Infection caused by coronavirus is coronavirus infection.

The term "novel coronavirus" means SARS-CoV-2 reported at the end of 2019 among coronaviruses. The coronavirus infection caused by SARS-CoV-2 is also referred to as COVID-19.

The term "a variant of novel coronavirus" means a virus with mutations in at least part of the genetic information of Wuhan-Hu-1 strain (Accession No. NC_045512), which is defined as a reference strain at the website of NCBI (https://www.ncbi.nlm.nih.gov/sars-cov-2/). The term "variant" used herein is a term that encompasses both "variant strain" and "variant species."

Variants of novel coronavirus include variants published in "Kansen/denpasei no zouka ya kougensei no henka ga kenen sareru shingata coronavirus (SARS-CoV-2) no shinki henikabu ni tsuite" (in Japanese) (Novel variant strains of novel coronavirus (SARS-CoV-2) of concern about increased infectivity/contagiosity and change in antigenicity) at the website of National Institute of Infectious Diseases (https://www.niid.go.jp/niid/ja/2019-ncov/2551-cepr/10743-covidl9-62.html). Examples thereof include alpha strain, beta strain, gamma strain, delta strain, (formerly) kappa strain, lambda strain, mu strain, and AY4.2.

A mutation in the genetic information may occur anywhere in the genome of SARS-CoV-2, and examples thereof include a mutation that occurs in a gene coding for a structural protein or a non-structural protein. Examples of the structural protein include S protein, M protein, E protein, and N protein. Examples of the non-structural protein include primases, RNA-dependent RNA polymerases, helicases, exonucleases, ribonucleases, methyl transferases, and proteases.

Examples of a variant with a mutation that has occurred in the gene coding for S protein of SARS-CoV-2 include B.1.1.7 (501YV1), B. 1.351 (501YV2), P. 1 (501YV3), B. 1.427 (20C/S:452R), and B.1.429 (20C/S:452R).

Examples of a variant with a mutation that has occurred in the gene coding for M protein or E protein, other structural proteins of SARS-CoV-2, include viruses that have mutations described in a reference document (Jakhmola S, Indari O, Kashyap D, Varshney N, Das A, Manivannan et al. Mutational analysis of structural proteins of SARS-CoV-2. Heliyon. 2021; 7:e06572).

A variant of novel coronavirus is a virus in which viral properties of SARS-CoV-2 have changed. Examples of changes in the viral properties mentioned herein include increased or decreased virulence, increased or decreased immune escaping ability, increased or decreased human-human infection efficiency, increased or decreased resistance to a coronavirus infection therapeutic drug, increased or decreased effectiveness of a novel coronavirus vaccine, and increased or decreased degree of viral proliferation.

A variant with increased or decreased virulence indicates a virus whose pathogenicity against the host has changed. A virus with an increased immune escaping ability indicates a virus that has changed so that it is not easily eliminated by immunity because of the difficulty for the host to recognize it as a foreign body. A virus with increased or decreased human-human infection efficiency indicates a virus whose infection efficiency has changed because of change in the ability of a host cell to bind to ACE2 or the like. A virus with increased or decreased resistance to a coronavirus infection therapeutic drug is a virus in which a therapeutic agent-acting site has changed in a structural protein or a non-structural protein of a virus. A virus for which the effectiveness of a novel coronavirus vaccine has been increased or decreased indicates a virus whose responsiveness to a neutralizing antibody acquired by the host from vaccination has increased or decreased. A virus with an increased or decreased degree of viral proliferation indicates a virus whose viral proliferation efficiency in a host cell has increased or decreased.

The following variants of novel coronavirus have been identified: B.1.1.7 (501Y.V1), B.1.351 (501YV2), P.1 (501YV3), B.1.427 (20C/S:452R), B.1.429 (20C/S:452R), B.1.526 (20C/S:484K), B.1.526.1 (20C), B.1.525 (20A/S:484K), P.2 (20J), B.1.617 (20A), B.1.617.1 (20A/S:154K), B.1.617.2 (20A/S:478K), B.1.617.3 (20A), B.1.1.316, B.1.617 + S:V382L, and experimentally or clinically isolated coronaviruses resistant to remdesivir.

The major amino acid mutation sites identified in the above-mentioned variants of novel coronavirus are as shown in Table 1 below.

[Table 1]

| Protein name | Amino acid mutation site | Action of mutation |
| --- | --- | --- |
| S protein | W152C, 152L | Involved in decreased responsiveness to neutralizing antibody |
| | V382L | Involved in decreased responsiveness to neutralizing antibody |
| | L452R | Involved in immune escaping |
| | E484K | May decrease effect of immunity or vaccine |
| | N501Y | May increase infection efficiency |
| | D614G | Increases proliferation efficiency and is involved in increased contagiosity |
| RNA-dependent RNA polymerase | F480L | Involved in resistance to remdesivir |
| | D484Y | Involved in resistance to remdesivir |
| | V557L | Involved in resistance to remdesivir |
| | E802A/D | Involved in resistance to remdesivir |

The variants of novel coronavirus also include unknown variants such as variants that have not been identified or have not emerged. Unknown variants can be identified at the website of CDC (https://www.cdc.gov/coronavirus/2019-ncov/cases-updates/variant-surveillance/variant-info.html), which is updated as needed.

The treatment means to reduce or improve one or more symptoms caused by a specific disease affecting a subject, and to delay the progression of the disease. In an embodiment of the present invention, treatment means, for example, to reduce or improve symptoms such as pyrexia, coughing, and pneumonia in a patient with infection caused by a variant of novel coronavirus, and, for example, to improve body temperature, percutaneous oxygen saturation (SpO$_2$), and chest image findings or to achieve negative testing for a variant of novel coronavirus.

Compound A or a salt thereof used in the present invention can be manufactured by methods known per se or by using those methods in combination. For example, Compound A can be manufactured according to a method described in International Publication No. WO 00/10569. It should be noted that Compound A has a tautomer 6-fluoro-3-oxo-3,4-dihydro-2-pyrazinecarboxamide.

Compound A or a salt thereof used in the present invention can be mixed with various pharmaceutical additives such as excipients, binders, disintegrants, disintegration inhibitors, anti-caking/anti-adhesive agents, lubricants, absorption or adsorption carriers, solvents, expanders, tonicity adjusting agents, dissolving aids, emulsifiers, suspending agents, thickeners, coating agents, absorption enhancers, gelling/solidifying enhancers, light stabilizers, preservatives, desiccating agents, emulsion, suspension, or dispersion stabilizers, coloring preventive agents, oxygen absorbers/antioxidants, taste masking/odor masking agents, coloring agents, foaming agents, defoaming agents, soothing agents, antistatic agents, and buffers or pH adjusters to formulate a drug product such as an oral agent (e.g., tablet, capsule, powder, granule, subtilized granule, pill, suspension, emulsions, liquid, and syrup), an injection, an eye drop, a nasal agent, or a percutaneous agent. It should be noted that the dosage form for a patient with infection caused by a variant of novel coronavirus is preferably an oral agent or an injection, more preferably an oral agent, yet more preferably a tablet.

The above-mentioned drugs are formulated by usual methods.

The method of administering Compound A is not particularly limited and is suitably decided depending on the dosage form, patient's age, sex, and other conditions, and severity of patient's symptom.

The dose of Compound A is suitably selected depending on a dosing regimen, patient's age, sex, disease form, other conditions, and the like. For example, the adult dose is 10 to 5000 mg or preferably 200 to 2400 mg of Compound A, which can be administered once a day or divided into several doses a day. Compound A can be administered several times or as a single dose until the desired treatment effect is achieved. Administration is typically monitored and can be repeated as necessary.

In the present invention, 1000 to 2400 mg of Compound A can be administered to an adult twice a day on Day 1, followed by administration of 400 to 1200 mg twice a day on Day 2 and beyond. Administration of 1600 mg of Compound A to an adult twice a day on Day 1, followed by 600 mg twice a day on Day 2 and beyond, and administration of 1800 mg of Compound A to an adult twice a day on Day 1, followed by 800 mg twice a day on Day 2 and beyond, are preferred, and administration of 1800 mg of Compound A twice a day on Day 1, followed by 800 mg twice a day on Day 2 and beyond, is more preferred.

As an interval between the two doses in one day, the second dose is preferably administered with an interval of at least 4 hours after the first dose, and the second dose is administered more preferably with an interval of at least 6 hours.

The administration period can be suitably decided depending on changes in symptoms over time and can be selected from maximum 5 days, 6 days, 7 days, 8 days, 9 days, 10 days, 11 days, 12 days, 13 days, 14 days, 15 days, 16 days, 17 days, 18 days, 19 days, 20 days, 21 days, and 22 days. Maximum 10 days, 13 days, 14 days, and 22 days are preferred, and maximum 13 days and 14 days are more preferred.

In the present invention, administration of Compound A or a salt thereof can include administration of a combination drug and/or combination therapy. A coronavirus infection therapeutic drug can be used as a combination drug and/or combination therapy. Example of a coronavirus infection therapeutic drug include the following drugs:

(1) Interferon;
(2) Nucleic acid analogue;
(3) Protease inhibitor;
(4) Furin convertase cleavage inhibitor;
(5) Reverse transcriptase inhibitor and/or other RNA polymerase inhibitor;
(6) Neuraminidase inhibitor;
(7) Angiotensin II receptor blocker;
(8) Endosome fusion inhibitor and/or endosome alkalinizer;
(9) AAK1, GAK, and clathrin A,B,C (endocytosis) inhibitor
(10) Hemagglutinin esterase inhibitor;
(11) Cytokine or inflammation inhibitor or modifier;
(12) Cathepsin B inhibitor;
(13) Cathepsin L inhibitor;
(14) Helicase nsp13 inhibitor;
(15) MBL2 gene agonist;
(16) TP53 inhibitor;
(17) Selective estrogen receptor modifier;
(18) Corticosteroid; and
(19) Other drugs.

Examples of the interferon include interferon $\alpha$-2A, interferon $\alpha$-2B, interferon $\alpha$-n1, interferon $\alpha$-n3, interferon $\beta$-1a, and interferon $\beta$-1b. It is sufficient to manufacture an interferon by using methods known per se in combination or to use a commercially available one.

Examples of the nucleic acid analogue include acyclovir, ganciclovir, ribavirin, and taribavirin. It is sufficient to manufacture a nucleic acid analogue by using methods known per se in combination or to use a commercially available one.

Examples of the protease inhibitor include indinavir, nelfinavir, saquinavir, camostat, lopinavir/ritonavir combination (product name, Kaletra), epigallocatechin gallate, kaempferol-7-glucoside, mycophenolic acid, darunavir, mercaptopurine, disulfiram, nafamostat, and PF-07321332. It is sufficient to manufacture a protease inhibitor by using methods known per se in combination or to use a commercially available one.

Examples of the furin convertase cleavage inhibitor include tenofovir disoproxil, dolutegravir, boceprevir, andrographolide, luteolin, and baicalein. It is sufficient to manufacture a furin convertase cleavage inhibitor by using methods known per se in combination or to use a commercially available one.

Examples of the reverse transcriptase inhibitor and/or the RNA polymerase inhibitor include remdesivir, sofosbuvir, dactinomycin, galidesivir, baloxavir marboxil, molnupiravir, sangivamycin, and AT-527. It is sufficient to manufacture a reverse transcriptase inhibitor and/or RNA polymerase inhibitor by using methods known per se in combination or to use a commercially available one.

Examples of the neuraminidase inhibitor include oseltamivir and zanamivir. It is sufficient to manufacture a neuraminidase inhibitor by using methods known per se in combination or to use a commercially available one.

Examples of the angiotensin II receptor blocker include valsartan, telmisartan, losartan, irbesartan, azilsartan, olmesartan, and emodin. It is sufficient to manufacture an angiotensin II receptor blocker by using methods known per se in combination or to use a commercially available one.

Examples of the endosome fusion inhibitor and/or endosome alkalinizer include baicalin, chloroquine, hydroxychloroquine, griffithsin, quinine, and lactoferrin. It is sufficient to manufacture an endosome fusion inhibitor and/or endosome alkalinizer by using methods known per se in combination or to use a commercially available one.

Examples of the AAK1, GAK, and clathrin A,B,C (endocytosis) inhibitor include baricitinib, sunitinib, erlotinib, fedratinib, gefitinib, and silibinin. It is sufficient to manufacture AAK1, GAK, and a clathrin A,B,C (endocytosis) inhibitor by using methods known per se in combination or to use a commercially available one.

Examples of the hemagglutinin esterase inhibitor include 3,4-dichloroisocoumarin. It is sufficient to manufacture a hemagglutinin esterase inhibitor by using methods known per se in combination or to use a commercially available one.

Examples of the cytokine or inflammation inhibitor or modifier include ligustrazine, statin, melatonin, eplerenone, and methylprednisolone. It is sufficient to manufacture a cytokine or inflammation inhibitor or modifier by using methods known per se in combination or to use a commercially available one.

Examples of the cathepsin B inhibitor include salvianolic acid B. It is sufficient to manufacture a cathepsin B inhibitor by using methods known per se in combination or to use a commercially available one.

Examples of the cathepsin L inhibitor include MOL736, chelidocystatin, astaxanthin, curcumin, and vitamin D. It is sufficient to manufacture a cathepsin L inhibitor by using methods known per se in combination or to use a commercially available one.

Examples of the helicase nsp13 inhibitor include valsartan, Bananin, Iodobananin, Vanillinbananin, Eubananin, and silvestrol. It is sufficient to manufacture a helicase nsp13 inhibitor by using methods known per se in combination or to use a commercially available one.

Examples of the MBL2 gene agonist include β-glucan and vitamin A. It is sufficient to manufacture an MBL2 gene agonist by using methods known per se in combination or to use a commercially available one.

Examples of the TP53 inhibitor include vitexin and gossypol. It is sufficient to manufacture a TP53 inhibitor by using methods known per se in combination or to use a commercially available one.

Examples of the selective estrogen receptor modifier include toremifene and equilin. It is sufficient to manufacture a selective estrogen receptor modifier by using methods known per se in combination or to use a commercially available one.

Examples of the corticosteroid include ciclesonide and dexamethasone. It is sufficient to manufacture a corticosteroid by using methods known per se in combination or to use a commercially available one.

Examples of other drugs include amantadine, foscarnet, triazavirin, umifenovir, rapamycin, everolimus, nitazoxanide, tizoxanide, Caraphenol A, ivermectin, VIR-2703, tocilizumab, bamlanivimab, etesevimab, casirivimab/imdevimab, AZD7422, VIR-7831, VIR-7832, and BI 767551. It is sufficient to manufacture amantadine, foscarnet, triazavirin, umifenovir, rapamycin, everolimus, nitazoxanide, tizoxanide, Caraphenol A, ivermectin, VIR-2703, tocilizumab, bamlanivimab, etesevimab, casirivimab/imdevimab, AZD7422, VIR-7831, VIR-7832, and BI 767551 by using methods known per se in combination or to use a commercially available one.

**EXAMPLES**

The present invention will be described below using Test examples, but the present invention is not limited to these.

Test example 1

A test of Compound A is performed using a viral infection cell model. Vero E6 cells are used as cells. Specifically, antiviral effect is evaluated by detecting cytopathic effect (CPE). It should be noted that, in addition to Vero E6 cells, Vero, Vero 76, CaCo-2, Hep G2, and Calu-3 cells and the like, to which a used virus exhibits susceptibility, can also be used for evaluation. In addition to the CPE assay, the antiviral effect can be evaluated by a viral antigen-antibody method or by real-time polymerase chain reaction (PCR) of virus RNA extracted from a virus culture supernatant.

The B.1.1.7 (501Y.V1) and B.1.351 (501YV2) strains, variants of novel coronavirus, are selected as RNA viruses.

(1) Vero E6 cell culture

Africa green monkey kidney Vero E6 cells that have been subcultured in a 10% bovine fetus serum-added Eagle MEM/kanamycin containing 60 μg/mL Eagle MEM/kanamycin medium at 37°C under the 5% carbon dioxide condition are removed from the culture broth by the trypsin-ethylenediaminetetraacetic acid method, and a suspension containing $2 \times 10^4$ cells per 100 μL of the medium is prepared and seeded on a 96-well plate. The Vero E6 cells are cultured overnight at 37°C under the 5% carbon dioxide condition and obtained as a monolayer.

(2) Infection with variants of novel coronavirus and addition of drugs

A 2% bovine fetus serum-added Eagle MEM/kanamycin medium is used as a test medium. The culture supernatant containing Vero E6 cells obtained in (1) is removed, and the following (A) or (B) is added to each well, and the mixture is cultured at 37°C for 1 to 2 hours under the 5% carbon dioxide condition:

(A) 100 μL of a solution of a variant of novel coronavirus prepared with a test medium to obtain a final multiplicity of infection of 0.1 to 10

(B) 100 μL of 0.5% DMSO-containing test medium that contains a combination of Compound A at concentrations two-fold the specified concentrations for each combination of the specified concentrations (drug):
Specified concentrations (μM) of Compound A:
0, 0.1, 0.3, 1, 3, 10, 30, 100, 300, 1000, 3000, 5000, 8000, 10,000

At 1 to 2 hours after infection, the culture supernatant is removed from each well, and 100 μL of 0.5% DMSO-containing test medium (drug) that contains Compound A at concentrations one-fold the specified concentrations is added. After the drug is added, the cells are cultured at 37°C for 2 to 3 days under the 5% carbon dioxide condition.

(3) Determination of cytopathic effect (CPE)

The CPE associated with proliferation of SARS-CoV-2 is determined by the following method.
After completion of culture, 25 μL of 100% formalin solution is added to each well to inactivate virus and immobilize cells. The mixture is left stand at room temperature for 2 hours or longer, the aqueous solution is removed, cells are gently washed with water, then 50 μL of 0.02% methylene blue solution is added to each well, and the mixture is left stand at room temperature for one hour. The aqueous solution is removed, and cells are gently washed with water and then air-dried. Then, absorbance (660 nm) is measured using a microplate reader. For a non-infected control, 100 μL of the test medium is added instead of the SARS-CoV-2 solution, the same operations as for the test group are performed, and the absorbance is measured.
A test is performed for two plates (one patient per plate) (eight patients for infected controls and non-infected controls). The value obtained by subtracting the absorbance of the infected control from the absorbance of the non-infected control is used as the value indicating complete inhibition of viral proliferation, and the CPE inhibition rate is calculated for each test by the following formula:

$$\text{CPE inhibition rate} = 100 \times (\text{[absorbance when Compound A is used]} -$$
$$\text{(absorbance of infected control])} / (\text{[absorbance of non-infected control)} - (\text{absorbance of}$$
$$\text{infected control])}$$

Microsoft Office Excel 2016 FORECAST function (linear regression) is used to calculate the 50% CPE inhibitory concentration.
The 50% inhibitory concentrations of Compound A against the B.1.1.7 (501Y.V1) and B.1.351 (501YV2) strains, variants of novel coronavirus, are calculated.
It is confirmed that Compound A is susceptible to the B.1.1.7 (501Y.V1) and B.1.351 (501YV2) strains, variants of novel coronavirus.

Test example 2

Variants of novel coronavirus, P.1 (501YV3), B.1.427 (20C/S:452R), B.1.429 (20C/S:452R), B.1.526 (20C/S:484K), B.1.526.1 (20C), B.1.525 (20A/S:484K), P.2(20J), B.1.617 (20A), B.1.617.1 (20A/S:154K), B.1.617.2 (20A/S:478K), B.1.617.3 (20A), B.1.1.316, B.1.617 + S:V382L, or an experimentally or clinically isolated coronavirus resistant to remdesivir are selected as RNA viruses, and the 50% inhibitory concentrations of Compound A are calculated using the same technique as in the above Test example 1 to demonstrate that Compound A is susceptible to various variants of novel coronavirus.

Test example 3

<Measurement of drug susceptibility>

The drug susceptibility of Compound A to novel coronavirus and variants of novel coronavirus was measured by a yield reduction assay. USA_WA1/2020 (A line) was selected as a wild strain of novel coronavirus, and the hCoV-19/England/204820464/2020 (B.1.1.7 line) and hCoV-19/Japan/TY7-503/2021 (P.1 line) strains were selected as variants of novel coronavirus. After viruses were proliferated in the presence of 3.2 to 1000 $\mu$M (common ratio 3) Compound A, the viral load at each concentration was measured. The 90% effective concentration (EC$_{90}$) was calculated from the obtained viral load.

<Viral proliferation>

Vero 76 cells suspended in a cell culture medium were seeded on a plate on the day before viral infection. The cells were cultured at 37°C under the 5% $CO_2$ condition, and the Vero 76 cells obtained as a monolayer were used for tests on the following day. A virus culture medium containing Compound A was added, and the mixture was pretreated at 37°C for one hour under the 5% $CO_2$ condition. After the medium was removed by suction, a culture medium containing Compound A and a virus solution (multiplicity of infection, 0.01) was added to each well at a predetermined final concentration, and the mixture was cultured at 37°C for 3 to 4 days under the 5% $CO_2$ condition. After completion of culture, the culture supernatant in each well was transferred to a fresh plate and stored at -80°C until virus was quantified.

<Quantification of virus and calculation of EC$_{90}$ value>

Vero 76 cells suspended in a cell culture medium were seeded on a plate on the day before viral infection. The virus culture broth obtained in the section <Viral proliferation> was diluted serially by common ratio of 10, and then the diluted viral solution was added to Vero 76 cells (n = 4), and the mixture was cultured at 37°C for 3 to 4 days under the 5% $CO_2$ condition. After completion of culture, the presence or absence of CPE in each well was examined, and then the 50% cell culture infectious dose (CCID$_{50}$/mL [log$_{10}$]) was calculated by the Reed-Muench method. In addition, the EC$_{90}$ value was calculated by regression analysis of the log$_{10}$ value of Compound A concentrations and the viral load (CCID$_{50}$/mL [log$_{10}$]).

The EC$_{90}$ values of Compound A against the USA_WA1/2020 (A line), hCoV-19/England/204820464/2020 (B.1.1.7 line), and hCoV-19/Japan/TY7-503/2021 (P.1 line) strains were 68 $\mu$M, 83 $\mu$M, and 190 $\mu$M, respectively.

Given that changes in the EC$_{90}$ value of Compound A against the hCoV-19/England/204820464/2020 (B.1.1.7 line) and hCoV-19/Japan/TY7-503/2021 (P.1 line) strains ranged from 1.2 to 2.8-fold as compared with that against USA_WA1/2020 (Aline), Compound A was found to exhibit drug effect against variant strains that was comparable to that against the wild strain of novel coronavirus.

Further, by the same method as described above using novel coronavirus hCoV-19/Japan/TY2-312-P1/2020 (B.1.1 line) as a reference strain, the drug susceptibility of Compound A was measured to the hCoV-19/Japan/QK002/2020 (B.1.1.7 line), hCoV-19/Japan/TY8-612/2021 (B.1.351 line), hCoV-19/Japan/TY7-501/2021 (P.1 line), hCoV-19/Japan/TY11-927-P1/2021 (B.1.617 line), and hCoV-19/Japan/TY11-330-P1/2021 (B.1.617.1 line) strains, variants of novel coronavirus. Of note, the concentration of Compound A present during viral proliferation was 100 to 1000 $\mu$M (common ratio 3.3), the duration of pretreatment after addition of Compound A was 3 hours, the multiplicity of infection of the viral solution added was 0.001, the duration of culture after addition of the viral solution was 40 hours, and Vero E6/TMPRSS2 cells were used. The test was performed in two parts.

The EC$_{90}$ values of Compound A against the above-mentioned reference strain and variant strains were 302 $\mu$M (Test 1) or 466 $\mu$M (Test 2), 359 $\mu$M (Test 1), 223 $\mu$M (Test 2), 306 $\mu$M (Test 2), 294 $\mu$M (Test 1), and 491 $\mu$M (Test 2). Given that the changes in the EC$_{90}$ value of Compound A against variant strains ranged from 0.48 to 1.2-fold as compared with against the reference strain, Compound A was found to exhibit a drug effect against variant strains that was comparable to that against the reference strain.

Test example 4

<Cell culture>

Vero E6 cells were seeded on a 6-well assay plate and incubated for approximately 4 hours before being treated with Compound A.

<Pretreatment and potency test>

Cells in each well were pretreated with a Compound A solution serially diluted to a concentration described later for approximately 4 hours and infected with novel coronavirus. SARS-2 WA1/2020 was used as a reference strain, and SARS-2-9017 (delta) and SARS-2 Omicron were used as variants of novel coronavirus. The potency of Compound A was assessed by setting concentrations of 7.81 to 1000 $\mu$M (with a common ratio of 2) per well on a plate. The multiplicity of infection of the viral solution added was 0.01, and the duration of culture after addition of the viral solution was 72 hours.

The culture supernatant solution was removed from each well containing infected cells, serially diluted with a common ratio of 10, and added to a monolayer of Vero E6 cells (n = 4), which were then cultured. After a clear CPE was observed, the 50% cell culture infectious dose ($CCID_{50}$/mL [$log_{10}$]) was calculated by the Reed-Muench method. In addition, the $EC_{90}$ value was calculated by regression analysis of the $log_{10}$ value of Compound A concentrations and the viral load ($CCID_{50}$/mL [$log_{10}$]).

Given that the $EC_{90}$ values of Compound A against the above-mentioned reference strain and variant strains were 125 to 250 $\mu$M (SARS-2 WA1/2020), 125 to 250 $\mu$M (SARS-2-9017 [delta]), and 62.5 to 125 $\mu$M (SARS-2 Omicron), Compound A was found to exhibit a drug effect against variant strains that was comparable to that against the reference strain.

These results indicate that Compound A is effective against variants of novel coronavirus.

## INDUSTRIAL APPLICABILITY

A therapeutic agent for a variant of novel coronavirus comprising 6-fluoro-3-hydroxy-2-pyrazinecarboxamide or a salt thereof as an active ingredient is useful in the field of pharmaceutical industry.

## Claims

1. A therapeutic agent for coronavirus infection caused by a variant of SARS-CoV-2, comprising 6-fluoro-3-hydroxy-2-pyrazinecarboxamide or a salt thereof as an active ingredient.

2. The therapeutic agent for coronavirus infection according to claim 1, wherein at least one of mutations in the genetic information of the Wuhan-Hu-1 strain has occurred in the gene coding for S protein in the variant.

3. The therapeutic agent for coronavirus infection according to claim 1, wherein at least one of mutations in the genetic information of the Wuhan-Hu-1 strain has occurred in the gene coding for M protein in the variant.

4. The therapeutic agent for coronavirus infection according to claim 1, wherein at least one of mutations in the genetic information of the Wuhan-Hu-1 strain has occurred in the gene coding for E protein in the variant.

5. The therapeutic agent for coronavirus infection according to claim 1, wherein at least one of mutations in the genetic information of the Wuhan-Hu-1 strain has occurred in the gene coding for N protein in the variant.

6. The therapeutic agent for coronavirus infection according to claim 1, wherein at least one of mutations in the genetic information of the Wuhan-Hu-1 strain has occurred in the gene coding for an RNA-dependent RNA polymerase in the variant.

7. The therapeutic agent for coronavirus infection according to claim 1, wherein at least one of mutations in the genetic information of the Wuhan-Hu-1 strain has occurred in the gene coding for an exonuclease in the variant.

8. The therapeutic agent for coronavirus infection according to claim 1, wherein at least one of mutations in the genetic information of the Wuhan-Hu-1 strain has occurred in the gene coding for a protease in the variant.

9. The therapeutic agent for coronavirus infection according to claim 1, wherein the variant is a virus with increased or decreased virulence as compared with that of SARS-CoV-2.

10. The therapeutic agent for coronavirus infection according to claim 1, wherein the variant is a virus with increased or decreased human-human infection efficiency as compared with that of SARS-CoV-2.

11. The therapeutic agent for coronavirus infection according to claim 1, wherein the variant is a virus with an increased immune escaping ability as compared with that of SARS-CoV-2.

12. The therapeutic agent for coronavirus infection according to claim 1, wherein the variant is a virus with increased or decreased resistance to a coronavirus infection therapeutic drug as compared with that of SARS-CoV-2.

13. The therapeutic agent for coronavirus infection according to claim 1, wherein the variant is a virus for which the effectiveness of a novel coronavirus vaccine has been increased or decreased as compared with for SARS-CoV-2.

14. The therapeutic agent for coronavirus infection according to claim 1, wherein the variant is B.1.1.7 (501YV1), B.1.351 (501YV2), P.1 (501YV3), B.1.427 (20C/S:452R), B.1.429 (20C/S:452R), B.1.526 (20C/S:484K), B.1.526.1 (20C), B.1.525 (20A/S:484K), P.2 (20J), B.1.617 (20A), B.1.617.1 (20A/S:154K), B.1.617.2 (20A/S:478K), B.1.617.3 (20A), B.1.1.316, B.1.617 + S:V382L, or an experimentally or clinically isolated coronavirus resistant to remdesivir.

## INTERNATIONAL SEARCH REPORT

International application No.

**PCT/JP2022/020028**

### A. CLASSIFICATION OF SUBJECT MATTER

*A61K 31/4965*(2006.01)i; *A61P 31/12*(2006.01)i; *A61P 31/14*(2006.01)i
FI:  A61K31/4965; A61P31/12; A61P31/14

According to International Patent Classification (IPC) or to both national classification and IPC

### B. FIELDS SEARCHED

Minimum documentation searched (classification system followed by classification symbols)

A61K31/4965; A61P31/12; A61P31/14

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| P, X | BUDHIRAJA, S. et al., Differentials in the characteristics of COVID-19 cases in Wave-1 and Wave-2 admitted to a network of hospitals in North India, medRxiv, 2021, pp. 1-18, DOI 10.1101/2021.06.24.21259438<br>in particular, pp. 6, 13 | 1-14 |
| P, A | PADHI, A. K. et al., Interface-based design of the favipiravir-binding site in SARS-CoV-2 RNA-dependent RNA polymerase reveals mutations conferring resistance to chain termination, FEBS Letters, 01 September 2021, 595, pp. 2366-2382<br>entire text | 1-14 |
| P, A | SHINADA, K. et al., Longitudinal Analysis of Neutralizing Potency against SARS-CoV-2 in the Recovered Patients after Treatment with or without Favipiravir, Viruses, 24 March 2022, 14, 670 (pp. 1-13), https://doi.org/10.3390/v14040670<br>entire text | 1-14 |
| P, A | WU, C.-R. et al., Structure genomics of SARS-CoV-2 and its Omicron variant: drug design templates for COVID-19, Acta Pharmacologica Sinica, 20 January 2022, 0, pp. 1-13, https://doi.org/10.1038/s41401-021-00851-w<br>entire text | 1-14 |

✓ Further documents are listed in the continuation of Box C.  ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **30 June 2022** | **12 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**INTERNATIONAL SEARCH REPORT**

| International application No. |
| --- |
| **PCT/JP2022/020028** |

### C.    DOCUMENTS CONSIDERED TO BE RELEVANT

| Category* | Citation of document, with indication, where appropriate, of the relevant passages | Relevant to claim No. |
| --- | --- | --- |
| P, A | RAMAN, R. et al., COVID-19: Unmasking Emerging SARS-CoV-2 Variants, Vaccines and Therapeutic Strategies, Biomolecules, 06 July 2021, 11, 993 (pp. 1-30), https://doi.org/10.3390/biom11070993 <br> entire text | 1-14 |
| P, A | ASSIS, L. C. et al., Theoretical insights into the effect of halogenated substituent on the electronic structure and spectroscopic properties of the favipiravir tautomeric forms and its implications for the treatment of COVID-19, RSC Adv., 2021, 11, pp. 35228-35244 <br> entire text | 1-14 |
| A | GHASEMNEJAD-BERENJI, M. et al., Favipiravir and COVID-19: A Simplified Summary, Drud Res, 11 November 2020, 71, pp. 166-170 <br> entire text | 1-14 |
| A | ZHOU, S. et al., β-D-N4-hydroxycytidine Inhibits SARS-CoV-2 Through Lethal Mutagenesis But Is Also Mutagenic To Mammalian Cells, The Journal of Infectious Diseases, 07 May 2021, 224, pp. 415-419 <br> entire text | 1-14 |
| A | DRIOUICH, J.-S. et al., Favipiravir antiviral efficacy against SARS-CoV-2 in a hamster model, nature communications, 19 March 2021, 12, 1735 (pp. 1-13), https://doi.org/10.1038/s41467-021-21992-w <br> entire text | 1-14 |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0010569 A


### Non-patent literature cited in the description

- **WANG C ; HORBY PW ; HAYDEN FG ; GAO GF.** A novel coronavirus outbreak of global health concern. *Lancet.,* 2020, vol. 395, 470-3
- Coronaviridae Study Group of the International Committee on Taxonomy of Viruses. The species Severe acute respiratory syndrome-related coronavirus: classifying 2019-nCoV and naming it SARS-CoV-2. *Nat Microbiol.,* 2020, vol. 5, 536-44
- Kokunai no hassei jokyo nado (in Japanese) (Situation of occurrences etc.). Ministry of Health, Labour and Welfar, 12 May 2021
- **GOWEN BB ; WONG MH ; JUNG KH ; SANDERS AB ; MENDENHALL M ; BAILEY KW et al.** In vitro and in vivo activities of T-705 against arenavirus and bunyavirus infections. *Antimicrob Agents Chemother.,* 2007, vol. 51, 3168-76
- **MENDENHALL M ; RUSSELL A ; SMEE DF ; HALL JO ; SKIRPSTUNAS R ; FURUTA Y et al.** *PLoS Negl Trop Dis.,* 2011, vol. 5, e1342
- **OESTEREICH L ; LUDTKE A ; WURR S ; RIEGER T ; MUNOZ-FONTELA C ; GUNTHER S.** Successful treatment of advanced Ebola virus infection with T-705 (favipiravir) in a small animal model. *Antiviral Res.,* 2014, vol. 105, 17-21
- **SZEMIEL et al.** vitro evolution of Remdesivir resistance reveals genome plasticity of SARS-CoV-2. *bioRxiv,* 2021, https://doi.org/10.1101/2021.02.01.429199
- **MARTINOT M ; JARY A ; FAFI-KREMER S ; LEDUCQ V ; DELAGREVERIE H ; GARNIER M et al.** Remdesivir failure with SARS-CoV-2 RNA-dependent RNA-polymerase mutation in a B-cell immunodeficient patient with protracted Covid-19. *Clin Infect Dis.,* 2020
- **AGOSTINI ML ; ANDRES EL ; SIMS AC ; GRAHAM RL ; SHEAHAN TP ; LU X et al.** Coronavirus Susceptibility to the Antiviral Remdesivir (GS-5734) Is Mediated by the Viral Polymerase and the Proofreading Exoribonuclease. *mBio.,* 2018, vol. 9, e00221-18
- **JAKHMOLA S ; INDARI O ; KASHYAP D ; VARSHNEY N ; DAS A, MANIVANNAN et al.** Mutational analysis of structural proteins of SARS-CoV-2. *Heliyon,* 2021, vol. 7, e06572